(19) Europäisches Patentamt
European Patent Office
Office européen des brevets

(11) Veröffentlichungsnummer: **0 467 207 A2**

(12) # EUROPÄISCHE PATENTANMELDUNG

(21) Anmeldenummer: **91111468.4**

(22) Anmeldetag: **10.07.91**

(51) Int. Cl.5: **C07D 473/00**, C07D 473/30, C07D 473/34, C07D 473/18

(30) Priorität: **18.07.90 CH 2387/90**

(43) Veröffentlichungstag der Anmeldung: **22.01.92 Patentblatt 92/04**

(84) Benannte Vertragsstaaten: **AT BE CH DE DK ES FR GB GR IT LI LU NL SE**

(71) Anmelder: **F. HOFFMANN-LA ROCHE AG
Postfach 3255
CH-4002 Basel(CH)**

(72) Erfinder: **Jaunin, Roland
28 Av. Parc-de-la-Rouvraie
CH-1018 Lausanne(CH)**

(74) Vertreter: **Kellenberger, Marcus, Dr. et al
Grenzacherstrasse 124 Postfach 3255
CH-4002 Basel(CH)**

(54) **Purinderivate.**

(57) Die Verbindungen der Formel

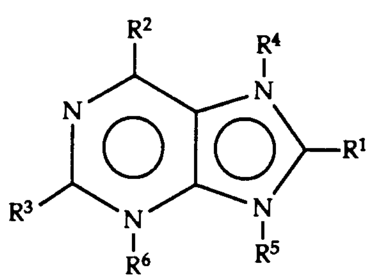

I

worin $R^1$, $R^2$, $R^3$, $R^4$, $R^5$ und $R^6$ die in Anspruch 1 angegebene Bedeutung besitzen, hemmen die Angiotensin II-Rezeptoren und können demnach in Form pharmazeutischer Präparate bei der Bekämpfung bzw. Verhütung von Bluthochdruck und Arteriosklerose verwendet werden. Sie können durch N-Biphenylmethylierung entsprechender unsubstituierter Purinderivate nach an sich bekannten Methoden hergestellt werden.

EP 0 467 207 A2

Die vorliegende Erfindung betrifft neue Purinderivate der allgemeinen Formel

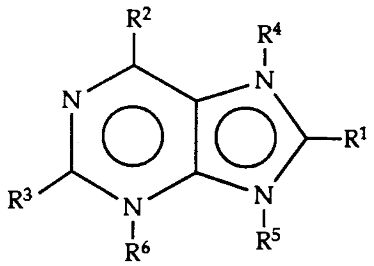

I

worin
$R^1$ Wasserstoff oder nieder-Alkyl;
$R^2$ Amino, Mono- oder Di-(nieder-alkyl)amino, $-SR^1$ oder $-OR^1$;
$R^3$ Wasserstoff, Amino, Mono- oder Di(nieder-alkyl)amino, $-SR^1$ oder $-OR^1$;
$R^4$, $R^5$ und $R^6$ einen Rest der Formel

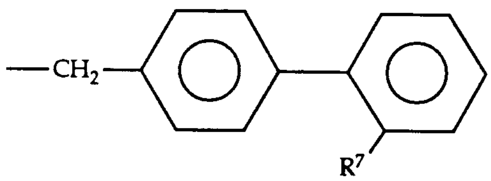

(a)

$R^7$ die Gruppe $-COOR^8$ oder einen Tetrazolylrest der Formel

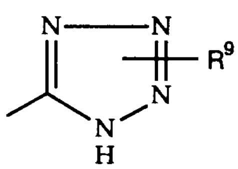

(b)

$R^8$ Wasserstoff, ein Alkali-, Erdalkali- oder Ammonium-Kation, nieder-Alkyl oder einen unter physiologischen Bedingungen abspaltbaren Rest; und

$R^9$ Wasserstoff oder ein Alkali-, Erdalkali- oder Ammonium-Kation bedeuten; wobei nur einer der Substituenten $R^4$, $R^5$ und $R^6$ anwesend ist und $R^2$ Amino oder Mono- oder Di-(nieder-alkyl)amino bedeutet, wenn $R^6$ anwesend ist.

Der hier verwendete Ausdruck "nieder-Alkyl" bezeichnet geradkettige oder verzweigte Alkylgruppen mit 1-7 C-Atomen, wie Methyl, Aethyl, Propyl, Isopropyl, Butyl und dessen Isomere. Beispiele von Alkali- und Erdalkalikationen sind $Na^+$, $K^+$, $Ca^{2+}$ und $Mg^{2+}$. Beispiele von Ammoniumionen sind $NH_4^+$ oder Trimethylammonium. Ein unter physiologischen Bedingungen abspaltbarer Rest ist z.B. Pivaloyloxymethyl.

Die Formel I umfasst somit Verbindungen der allgemeinen Formeln

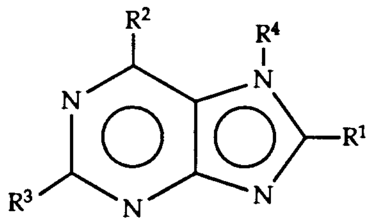

I a

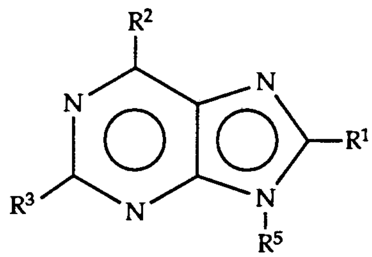

I b

und

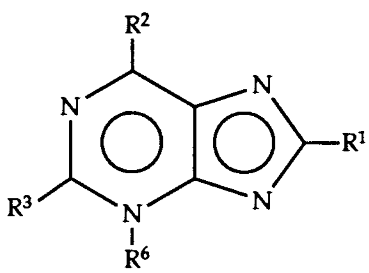

I c

und Tautomere davon.

Bevorzugte Verbindungen der Formel I sind solche, in denen $R^7$ einen Tetrazolylrest (b) bedeutet.

Weiterhin sind solche Verbindungen der Formel I bevorzugt, in denen $R^1$ nieder-Alkyl, insbesondere n-Propyl oder n-Butyl, bedeutet.

Repräsentative Vertreter von Verbindungen der Formel I sind:

4'-[(2-Amino-1,6-dihydro-6-oxo-9H-purin-9-yl)methyl]-2-biphenylcarbonsäure,
4'-[(2-Amino-1,6-dihydro-6-oxo-7H-purin-7-yl)methyl]-2-biphenylcarbonsäure,
4'-[(8-Butyl-1,6-dihydro-6-oxo-9H-purin-9-yl)methyl]-2-biphenylcarbonsäure,
4'-[(1,6-Dihydro-6-oxo-8-propyl-9H-purin-9-yl)methyl]-2-biphenylcarbonsäure,
4'-[(8-Butyl-6-methoxy-7H-purin-7-yl)methyl]-2-biphenylcarbonsäure,
4'-[(6-Amino-8-butyl-3H-purin-3-yl)methyl]-2-biphenylcarbonsäure,
4'-[(1,6-Dihydro-6-oxo-7H-purin-7-yl)methyl]-2-biphenylcarbonsäure,
Methyl 4'-[(6-amino-8-butyl-9H-purin-9-yl)methyl]-2-biphenylcarboxylat,
Methyl 4'-[(6-amino-8-butyl-3H-purin-3-yl)methyl]-2-biphenylcarboxylat,
Methyl 4'-[(6-methoxy-8-propyl-9H-purin-9-yl)methyl]-2-biphenylcarboxylat,
Methyl 4'-[(6-methoxy-8-propyl-7H-purin-7-yl)methyl]-2-biphenylcarboxylat,
Methyl 4'-[(1,6-dihydro-6-oxo-7H-purin-7-yl)methyl]-2-biphenylcarboxylat,
Methyl 4'-[(2-acetamido-6-[(diphenylcarbamoyl)oxy]-9H-purin-9-yl)methyl]-2-biphenylcarboxylat und
Methyl 4'-[(2-acetamido-6-[(diphenylcarbamoyl)oxy]-7H-purin-7-yl)methyl]-2-biphenylcarboxylat.

Besonders bevorzugte Verbindungen der Formel I sind:

4'-[(8-Butyl-1,6-dihydro-6-oxo-7H-purin-7-yl)methyl]-2-biphenylcarbonsäure,
4'-[(1,6-Dihydro-6-oxo-8-propyl-7H-purin-7-yl)methyl]-2-biphenylcarbonsäure,
4'-[(8-Butyl-6-methoxy-9H-purin-9-yl)methyl]-2-biphenylcarbonsäure,
4'-[(6-Amino-8-butyl-9H-purin-9-yl)methyl]-2-biphenylcarbonsäure,
8-Butyl-1,9-dihydro-9-[[2'-(1H-tetrazol-5-yl)-4-biphenylyl]methyl]-6H-purin-6-on und
8-Butyl.1,7-dihydro-7-[[2'-(1H-tetrazol-5-yl)-4-biphenylyl]methyl]-6H-purin-6-on.

Die Verbindungen der Formel I können erfindungsgemäss dadurch hergestellt werden, dass man eine Verbindung der allgemeinen Formel

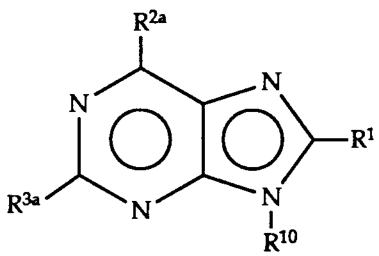

II

worin $R^{2a}$ einen wie weiter oben definierten Rest $R^2$ bedeutet, wobei die Amino-, Mercapto- oder Hydroxygruppe in geschützter Form vorliegen kann; $R^{3a}$ einen wie weiter oben definierten Rest $R^3$ bedeutet, wobei die Amino-, Mercapto- oder Hydroxygruppe in geschützter Form vorliegen kann; $R^{10}$ Wasserstoff oder ein N,N- oder N,O-Ketal bzw. -Aminal bedeutet; und $R^1$ die weiter oben angegebene Bedeutung hat;
mit einer Verbindung der allgemeinen Formel

Z-X III

worin X eine Abgangsgruppe und Z einen Rest der Formel

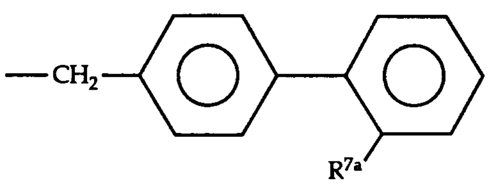

(a')

$R^{7a}$ die Gruppe $-COOR^{8a}$ oder einen Tetrazolylrest der Formel

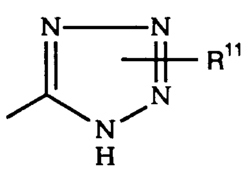

(b')

$R^{8a}$ nieder-Alkyl oder einer unter physiologischen Bedingungen abspaltbaren Rest und $R^{11}$ eine Schutzgruppe bedeuten, umsetzt, aus dem Reaktionsprodukt gegebenenfalls anwesende Schutzgruppen abspaltet und erwünschtenfalls eine Estergruppe $R^{8a}$ verseift, und eine Aether- oder Thioäthergruppe $R^2$ spaltet und/oder erwünschtenfalls das Reaktionsprodukt in ein Salz überführt.

Die Umsetzung einer Verbindung der Formel II mit einer Verbindung der Formel III wird zweckmässig in einem inerten organischen Lösungsmittel in Gegenwart einer Base vorgenommen. Beispiele geeigneter inerter organischer Lösungsmittel sind niedere aliphatische Alkohole, wie Methanol oder Aethanol.

Beispiele von Basen, die in der Reaktion eingesetzt werden können, sind Alkalimetallalkoholate, wie Natriummethylat oder -äthylat. Die Reaktionstemperatur ist nicht kritisch, vorzugsweise wird die Reaktion bei Raumtemperatur durchgeführt. Beispiele von Abgangsgruppen X in der Verbindung der Formel III sind Halogen, insbesondere Brom, Tosyloxy und Mesyloxy. Beispiele von Schutzgruppen $R^{11}$ sind Trityl oder Benzyloxymethyl.

Die Umsetzung der Verbindungen der Formel II mit Verbindungen der Formel III liefert Verbindungen der Formel I, in denen gegebenenfalls anwesende Amino-, Mercapto- oder Hydroxygruppen in geschützter Form vorliegen können und in denen ein gegebenenfalls anwesender Tetrazolylrest in geschützter Form vorliegt. Diese Schutzgruppen können in an sich bekannter Weise abgespalten werden. Die Abspaltung

einer Tritylgruppe $R^{11}$ kann z.B. durch Behandlung mit Basen, wie wässrig-alkoholischer Natronlauge, erfolgen, wobei eine gegebenenfalls anwesende niedere Alkyläther- oder Thioäthergruppe $R^2$ und $R^3$ ebenfalls abgespalten wird.

Eine Estergruppe $R^8$ und eine Aether- oder Thioäthergruppe $R^2$ oder $R^3$ kann in an sich bekannter Weise, z.B. durch Behandlung mit Basen, wie wässrig-alkoholischer Natronlauge, verseift werden.

Beispiele von Schutzgruppen für Aminogruppen sind Acylreste wie Acetyl; Beispiele für Hydroxyschutzgruppen sind Alkyl- oder Acylgruppen, wie Methyl oder Acetyl. Ein N,O-Aminal ist beispielsweise ein Zuckerrest, wie 1-Ribosyl.

Die Verbindungen der Formel I sind Hemmer der Angiotensin II-Rezeptoren und können zur Behandlung des Bluthochdrucks verwendet werden. Weiterhin hemmen die Verbindungen der Formel I die Migration und Proliferation von Zellen der vaskulären glatten Muskulatur und verhüten proliferative arteriosklerotische Läsionen. Die Verbindungen der Formel I können daher zur Prophylaxe der Arteriosklerose, insbesondere nach Bypass-Operationen oder Angioplastie, sowie zur Behandlung von Patienten mit progressiver Arteriosklerose verwendet werden.

Die Hemmwirkung auf Angiotensin II-Rezeptoren kann in vitro mittels eines Radiorezeptor-Bindungstests mit Hundenebennieren-Membranen ermittelt werden.

Hundenebennieren-Membranen werden für jeden Test frisch hergestellt. Die Nebennieren werden in 250 mM Saccharose-Lösung homogenisiert und 10 Minuten bei 4000 gav zentrifugiert. Der Ueberstand wird delipidiert und 30 Minuten bei 4000 gav rezentrifugiert. Das resultierende Sediment wird in einem Volumen Testpuffer (50 mM Tris/HCl, pH 7.4, 25 MnCl2, 1 mM EDTA, 0,5% w/v BSA, O,O1 % w/v NaN3) rehomogenisiert, so dass die Membranprotein-Konzentration etwa 1 mg/ml beträgt.

Für den Bindungstest werden 100 µl Nebennieren-Membransuspension mit 50000 cpm (3(125 J) Tyr)-Angiotensin II in Gegenwart von 5 µl Dimethylsulfoxid 20 Minuten bei 25° C unter Schütteln inkubiert. Die Testsubstanzen werden in Dimethylsulfoxid gelöst und in Konzentrationen von $10^{-9}$ M bis $10^{-4}$ M in diesem System getestet. Die unspezifische Bindung von (3(125 J)Tyr) Angiotension II wird in Gegenwart von 1 µM nicht jodiertem Angiotensin II bestimmt. Filtrieren eines Aliquots von 180 µl über eine Whatman Membran beendet die Inkubation. Die Membran wird noch zweimal mit jeweils 180 µl Testpuffer gewaschen. Membrangebundene Radioaktivität wird in einem Packard Gamma-Counter gemessen.

Die Resultate werden als IC-50 Werte angegeben, welche diejenigen Konzentrationen der Hemmer im Testsystem bezeichnen, die die spezifische Bindung von (3(125 J)Tyr) Angiotensin II an seinen Rezeptor um 50 % hemmt. IC-50 Werte werden aus den Hemmkurven (Prozent Bindung gegen decadischen Logarithmus der Hemmstoff-Konzentration) ermittelt. Alle Einzelwerte sind Mittelwerte aus Dreifachbestimmungen. Die angegebenen IC-50 Werte sind Mittelwerte aus wenigstens drei unabhängigen Versuchen. Bei der Berechnung der spezifischen Bindung von (3(125 J)Tyr) Angiotensin II wird die unspezifische Bindung jeweils subtrahiert.

Die mit diesem Test mit Verbindungen der Formel I erhaltenen Resultate sind in der folgenden Tabelle zusammengefasst:

## Tabelle I

| Verbindung | IC-50 (μM) |
|---|---|
| 4'-[(1,6-Dihydro-6-oxo-8-propyl-7H-purin-7-yl)methyl]-2-biphenylcarbonsäure | 0,365 |
| 4'-[(8-Butyl-6-methoxy-9H-purin-9-yl)methyl]-2-biphenylcarbonsäure | 0,458 |
| 4'-[(8-Butyl-1,6-dihydro-6-oxo-7H-purin-7-yl)methyl]-2-biphenylcarbonsäure | 0,167 |
| 4'-[(6-Amino-8-butyl-9H-purin-9-yl)methyl]-2-biphenylcarbonsäure | 0,534 |
| 8-Butyl-1,9-dihydro-9-[[2'-(1H-tetrazol-5-yl)-4-biphenylyl]-methyl]-6H-purin-6-on | 0,0955 |
| 8-Butyl-1,7-dihydro-7-[[2'-(1H-tetrazol-5-yl)-4-biphenylyl]-methyl]-6H-purin-6-on | 0,0658 |

Die Verbindungen können in Form üblicher galenischer Präparate enteral, parenteral oder topisch verabreicht werden. Für die enterale Applikation eignen sich z.B. Mittel in Form von Tabletten, Kapseln, Sirupen, Suspensionen, Lösungen und Suppositorien. Für die parenterale Applikation sind Mittel in Form von Infusions- oder Injektionslösungen geeignet.

Die Dosierungen, in denen die Präparate verabreicht werden, können je nach Anwendungsart und Anwendungsweg sowie nach den Bedürfnissen der Patienten variieren.

Im allgemeinen kommen beim Erwachsenen Dosierungen von etwa 0,5-500 mg/kg, vorzugsweise 1-100 mg/kg Körpergewicht pro Tag in Betracht.

Die Präparate können inerte oder auch pharmakodynamisch aktive Zusätze enthalten. Tabletten oder Granulate z.B. können eine Reihe von Bindemitteln, Füllstoffen, Trägersubstanzen oder Verdünnungsmitteln enthalten. Flüssige Präparate können beispielsweise in Form einer sterilen, mit Wasser mischbaren Lösung vorliegen. Kapseln können neben dem Wirkstoff zusätzlich ein Füllmaterial oder Verdickungsmittel enthalten. Des weiteren können geschmacksverbessernde Zusätze, sowie die üblicherweise als Konservierungs-, Stabilisierungs-, Feuchthalte- und Emulgiermittel verwendeten Stoffe, ferner auch Salze zur Veränderung des osmotischen Druckes, Puffer und andere Zusätze vorhanden sein.

Die vorstehend erwähnten Trägersubstanzen und Verdünnungsmittel können aus organischen oder anorganischen Stoffen, z.B. aus Wasser, Gelatine, Milchzucker, Stärke, Magnesiumstearat, Talkum, Gummi arabicum, Polyalkylenglykolen und dergleichen bestehen. Voraussetzung ist, dass alle bei der Herstellung der Präparate verwendeten Hilfsstoffe untoxisch sind.

Zur topischen Anwendung werden die Wirkstoffe zweckmässig in Form von transdermalen Applikationssystemen angewandt.

Die Erfindung wird durch die nachstehenden Beispiele weiter erläutert:

Beispiel 1

5,24 g 2-Acetamido-6-[(diphenylcarbamoyl)oxy]-9H-purin [Can. J. Chem. 65, 1436(1987)] wurden in 90 ml abs. Methanol suspendiert. Bei Raumtemperatur wurde eine Lösung von 13,5 mMol Natriummethylat in

9 ml abs. Methanol zugegeben. Die Lösung wurde 1 Stunde bei Raumtemperatur gerührt, das Methanol bei 35-40 °C unter Luft- und Feuchtigkeits-Abschluss eingeengt, der Rückstand in 18 ml Dimethylformamid aufgenommen, mit einer Lösung von 5,0 g (16,5 mMol) Methyl 4'-brommethylbiphenyl-2-carboxylat [EP 253'310] in 10 ml abs. Dimethylformamid versetzt und 4 Stunden bei 40 °C gerührt. Das Lösungsmittel wurde im Vakuum eingeengt, und der Rückstand chromatographiert ($SiO_2$/Essigester:Methanol 9:1 v/v). Man erhielt nach Umkristallisieren aus Essigester Methyl 4'-[(2-acetamido-6-[(diphenylcarbamoyl)oxy]-9H-purin-9-yl)methyl]-2-biphenylcarboxylat (weisse Kristalle, Smp. 186-188 °C) und Methyl 4'-[(2-acetamido-6-[-(diphenylcarbamoyl)oxy]-7H-purin-7-yl)methyl]-2-biphenylcarboxylat (weisse Kristalle, Smp. 175-177 °C).

Beispiel 2

a) 0,8 g Methyl 4'-[(2-acetamido-6-[(diphenylcarbamoyl)oxy]-9H-purin-9-yl)methyl]-2-biphenylcarboxylat wurde in 30 ml Aethanol und 15 ml 2N NaOH/$H_2O$ gelöst. Die Lösung wurde 18 Stunden rückflussiert, abgekühlt und mit konz. HCl auf pH 2,0 bei 0-5 °C gestellt. Der Niederschlag wurde abgenutscht, mit Wasser und Aether gewaschen. Das Filtrat wurde im Vakuum bei 40 °C eingeengt. Der Niederschlag wurde wie oben gewaschen, und das gesamte Produkt aus 1N HCl umkristallisiert. Man erhielt 4'-[(2-Amino-1,6-dihydro-6-oxo-9H-purin-9-yl)methyl]-2-biphenylcarbonsäure-hydrochlorid als weisse Kristalle vom Schmelzpunkt >250 °C.

b) In analoger Weise wurde aus Methyl 4'-[(2-acetamido-6-[(diphenylcarbamoyl)oxy]-7H-purin-7-yl)-methyl]-2-biphenylcarboxylat das 4'-[(2-Amino-1,6-dihydro-6-oxo-7H-purin-7-yl)methyl]-2-biphenylcarbonsäure-hydrochlorid als weisse Kristalle vom Schmelzpunkt >250 °C erhalten.

Beispiel 3

In Analogie zu Beispiel 1 wurde aus 5,4 g Inosin und 7,6 g Methyl 4'-brommethylbiphenyl-2-carboxylat (EP 253'310) das Methyl 4'-[(1,6-dihydro-6-oxo-7H-purin-7-yl)methyl]-2-biphenylcarboxylat in Form weisser Kristalle vom Schmelzpunkt 209-211 °C hergestellt.

Beispiel 4

a) 1,0 g (2,77 mMol) Methyl 4'-[(1,6-dihydro-6-oxo-7H-purin-7-yl)methyl]-2-biphenylcarboxylat wurde in 20 ml Methanol und 5 ml konz. Ammoniaklösung gelöst. 10 ml 1N NaOH-Lösung wurde zugegeben, und die Mischung 1 Stunde rückflussiert. Nach Abdestillieren der Lösungsmittel wurde der Rückstand in 10 ml $H_2O$ gelöst und mit 10 ml 1N HCl-Lösung neutralisiert. Der Niederschlag wurde abgenutscht, mit Wasser gewaschen, im Vakuum getrocknet, dann in ca. 20 ml 2N Ammoniaklösung gelöst und filtriert. Das Filtrat wurde im Vakuum lyophilisiert. Man erhielt 4'-[(1,6-Dihydro-6-oxo-7H-purin-7-yl)methyl]-2-biphenylcarbonsäure-ammoniumsalz als weisses Pulver, Smp. 100 °C (Zerstörung mit $NH_3$ Entwicklung).

Beispiel 5

Analog zu Beispiel 1 wurden aus 3,10 g 8-Butyl-6-methoxy-9H-purin und 5,34 g 4'-Brommethylbiphenyl-2-carboxylat (EP 253'310) das Methyl 4'-[(8-butyl-6-methoxy-9H-purin-9-yl)methyl]-2-biphenylcarboxylat und das Methyl 4'-[(8-butyl-6-methoxy-7H-purin-7-yl)methyl]-2-biphenylcarboxylat als leicht gelbe Oele erhalten.

Das als Ausgangsmaterial eingesetzte Purin kann wie folgt hergestellt werden:

In analoger Weise zu J.C.S. Perkin I, 1855 (1973) wurde aus 36,0 g 4,5-Diamino-6-hydroxy-pyrimidin-hemisulfat (Aldrich) das 5,6-Bis(valerylamino)pyrimidin-4(3H)-on, Schmelzpunkt 220-230 °C, daraus das 6-Amino-5-valerylaminopyrimidin-4(3H)-on, Schmelzpunkt >250 °C und schliesslich das 8-Butyl-2-chlor-9H-purin, Schmelzpunkt 75-77 °C erhalten.

1,22 g Natrium wurde in 37 ml abs. Methanol gelöst. 37 ml Dimethylformamid wurden zugegeben, und das Methanol unter Feuchtigkeitsausschluss unter Vakuum entfernt. Es wurde auf 0 °C abgekühlt, 5,0 g 8-Butyl-2-chlor-9H-purin zugegeben und 38 Stunden bei 90 °C gerührt. Die Lösung wurde auf 0 °C abgekühlt, und der pH durch Zusatz von 2N HCl-Lösung auf 7,0 gestellt. Der Niederschlag wurde filtriert, mit Wasser gewaschen und im Vakuum getrocknet. Das Produkt wurde mittels Chromatographie ($SiO_2$, MeOH:Essigester 1:4 v/v) gereinigt. Man erhielt 8-Butyl-6-methoxy-9H-purin als weisse Kristalle vom Schmelzpunkt 156-158 °C.

Beispiel 6

In Analogie zu Beispiel 1 wurden aus 2,90 g 6-Methoxy-8-propyl-9H-purin und 5,34 g 4'-Brommethylbiphenyl-2-carboxylat [EP 253'310] das Methyl 4'-[(6-methoxy-8-propyl-9H-purin-9-yl)methyl]-2-biphenylcarboxylat (Smp. 82-84 ° C) und das Methyl 4'-[(6-methoxy-8-propyl-7H-purin-7-yl)methyl]-2-biphenylcarboxylat (Smp. 147-150 ° C) als weisse Kristalle erhalten.

Das als Ausgangsmaterial eingesetzte Purin wurde wie im Anschluss an Beispiel 5 beschrieben ausgehend von 4,5-Diamino-6-hydroxypyrimidinhemisulfat über 5,6-Bis(butyrylamino)pyrimidin-4(3H)-on, 6-Amino-5-butyrylaminopyrimidin-4(3H)-on, Schmelzpunkt >250 ° C, 2-Chlor-8-propyl-9H-purin, Schmelzpunkt 115-117 ° C hergestellt. Man erhielt 6-Methoxy-8-propyl-9H-purin vom Schmelzpunkt 154-156 ° C.

Beispiel 7

3,70 g Methyl 4'-[(8-butyl-6-methoxy-9H-purin-9-yl)methyl]-2-biphenylcarboxylat wurden in 35 ml MeOH gelöst, mit 30 ml 1,0N NaOH versetzt und 1,5 Stunden unter Rückfluss gesetzt. Die Mischung wurde bei 5-10 ° C mit 30 ml 1,0N HCl versetzt. Der Niederschlag wurde in Essigester gelöst, und die organische Phase mit Wasser gewaschen. Nach Einengen der Lösungsmittel wurde der Rückstand mittels Chromatographie ($SiO_2$, Essigester:Methanol 4:1 v/v) gereinigt. Man erhielt 4'-[(8-Butyl-6-methoxy-9H-purin-9-yl)methyl]-2-biphenylcarbonsäure, Smp. >250 ° C.

In analoger Weise wurden erhalten: aus Methyl 4'-[(8-butyl-6-methoxy-7H-purin-7-yl)methyl]-2-biphenylcarboxylat die 4'-[(8-Butyl-6-methoxy-7H-purin-7-yl)methyl]-2-biphenylcarbonsäure. Weisse Kristalle, Smp. >240 ° C.

Beispiel 8

a) 0,6 g 4'-[(8-Butyl-6-methoxy-9H-purin-9-yl)methyl]-2-biphenylcarbonsäure wurden in 20 ml abs. Methanol gelöst, mit 20 ml 1N NaOH-Lösung versetzt und 15 Stunden rückflussiert. Die Mischung wurde heiss filtriert, auf 0 ° C gekühlt und mit 13 ml Methylenchlorid versetzt. Die trübe Lösung wurde langsam bei 0-5 ° C mit 2N HCl-Lösung auf pH 8,5 gestellt. Nach Abdestillieren von Methanol und Methylenchlorid im Vakuum und Abkühlen wurde das Produkt abgenutscht, in $H_2O$ gelöst, die Lösung filtriert und mit 0,5N HCl-Lösung auf pH 5,2 gestellt. Die Kristalle wurden abgenutscht, mit Wasser gewaschen und im Vakuum getrocknet. Man erhielt 4'-[(8-Butyl-1,6-dihydro-6-oxo-9H-purin-9-yl)methyl]-2-biphenylcarbonsäure. Weisse Kristalle, Smp. >250 ° C.

b) In analoger Weise wurden erhalten: aus 4'-[(8-Butyl-6-methoxy-7H-purin-7-yl)methyl]-2-biphenylcarbonsäure die 4'-[(8-Butyl-1,6-dihydro-6-oxo-7H-purin-7-yl)methyl]-2-biphenylcarbonsäure. Weisse Kristalle, Smp. >250 ° C.

Beispiel 9

In Analogie zu Beispiel 8 wurden aus Methyl 4'-[(6-methoxy-8-propyl-9H-purin-9-yl)methyl]-2-biphenylcarboxylat die 4'-[(1,6-Dihydro-6-oxo-8-propyl-9H-purin-9-yl)methyl]-2-biphenylcarbonsäure, Smp. 190-192 ° C (Zers.), und aus Methyl 4'-[(6-methoxy-8-propyl-7H-purin-7-yl)methyl]-2-biphenylcarboxylat die 4'-[(1,6-Dihydro-6-oxo-8-propyl-7H-purin-7-yl)methyl]-2-biphenylcarbonsäure, Smp. >250 ° C, erhalten.

Beispiel 10

In Analogie zu Beispiel 1 wurden aus 2,88 g 8-Butyl-adenin [J. Am. Chem. Soc. 105, 2050 (1983)] und 5,4 g 4'-Brommethylbiphenyl-2-carboxylat das Methyl 4'-[(6-amino-8-butyl-9H-purin-9-yl)methyl]-2-biphenylcarboxylat, Schmelzpunkt 204-206 ° C, und das Methyl 4'-[(6-amino-8-butyl-3H-purin-3-yl)methyl]-2-biphenylcarboxylat, Schmelzpunkt 174-176 ° C, erhalten.

Beispiel 11

0,6 g (14,4 mMol) Methyl 4'-[(6-amino-8-butyl-9H-purin-9-yl)methyl]-2-biphenylcarboxylat wurde in 10 ml Methanol gelöst, mit 5,0 ml 1N NaOH versetzt und 1,5 Stunden rückflussiert. Die Lösung wurde filtriert, auf 0-5 ° C abgekühlt, tropfenweise mit 5,0 ml 1N HCl-Lösung versetzt und über Nacht stehen gelassen. Das Produkt wurde abgenutscht, mit Wasser und Aether gewaschen und im Vakuum getrocknet. Man erhielt 4'-[(6-Amino-8-butyl-9H-purin-9-yl)methyl]-2-biphenylcarbonsäure. Weisse Kristalle, Smp. 250-253 ° C.

Beispiel 12

In analoger Weise zu Beispiel 11 wurde aus Methyl 4'-[(6-amino-8-butyl-3H-purin-3-yl)methyl]-2-biphenylcarboxylat die 4'-[(6-Amino-8-butyl-3H-purin-3-yl)methyl]-2-biphenylcarbonsäure, Schmelzpunkt >260 °C, erhalten.

Beispiel 13

In Analogie zu Beispiel 1 wurden aus 3,1 g 8-Butyl-6-methoxy-9H-purin und 9,8 g 5-[4'-Brommethylbiphenyl-2-yl]-2-triphenylmethyltetrazol das 8-Butyl-6-methoxy-9-[[2'-(triphenylmethyltetrazol-5-yl)-4-biphenylyl]methyl]purin als Oel, und das 8-Butyl-6-methoxy-7-[[2'-(triphenylmethyltetrazol-5-yl)-4-biphenylyl]methyl]purin als weisse Kristalle, Smp. 161-163 °C, erhalten.

1,0 g (1,50 mMol) 8-Butyl-6-methoxy-9-[[2'-(triphenylmethyltetrazol-5-yl)-4-biphenylyl]methyl]purin wurde in 20 ml Methanol gelöst, mit 20,0 ml NaOH (1,0N/$H_2O$) versetzt und 20 Stunden unter Rückfluss gesetzt. Das Methanol wurde im Vakuum eingeengt. Der pH wurde bei 0-5 °C mit 1,0N HCl-Lösung auf 4,9 gestellt. Das auskristallisierte Produkt wurde filtriert, mit Wasser gewaschen und im Vakuum getrocknet. Man erhielt das 8-Butyl-1,9-dihydro-9-[[2'-(1H-tetrazol-5-yl)-4-biphenylyl]methyl]-6H-purin-6-on, Schmelzpunkt 203-205 °C als weisse Kristalle.

Analog wurde aus 8-Butyl-6-methoxy-7-[[2'-(triphenylmethyltetrazol-5-yl)-4-biphenylyl]methyl]purin das 8-Butyl-1,7-dihydro-7-[[2'-(1H-tetrazol-5-yl)-4-biphenylyl]methyl]-6H-purin-6-on, Schmelzpunkt 220-224 °C (Zers.), erhalten.

Das als Ausgangsmaterial eingesetzte Tetrazol kann wie folgt hergestellt werden:

55,0 g 5-[4'-Methylbiphenyl-2-yl]tetrazol [EP 291'969] wurden in 700 ml abs. Methylenchlorid gegeben. 30,8 g (40,5 ml) N-Aethyldiisopropylamin wurden zugesetzt. Bei Raumtemperatur wurden 64,8 g Triphenylmethylchlorid zugegeben, und das Gemisch 28 Stunden bei Raumtemperatur gerührt. Die Lösungsmittel wurden im Vakuum eingeengt, der Rückstand mittels Chromatographie ($SiO_2/CH_2Cl_2$) gereinigt, und das 5-[4'-Methylbiphenyl-2-yl]-2-triphenylmethyltetrazol aus Diäthyläther umkristallisiert.

91,5 g 5-[4'-Methylbiphenyl-2-yl]-2-triphenylmethyltetrazol, 36,2 g N-Bromsuccinimid und 1,92 g Dibenzoylperoxid wurden in 480 ml Tetrachlorkohlenstoff während 48 Stunden unter Rückfluss erhitzt und dann abgekühlt. Der Succinimid-Niederschlag wurde abfiltriert und mit Tetrachlorkohlenstoff gewaschen. Das Filtrat wurde eingeengt, der Rückstand mit $CH_2Cl_2$ chromatographiert und das 5-[4'-Brommethylbiphenyl-2-yl]-2-triphenylmethyltetrazol aus Hexan umkristallisiert. Smp. 128-132 °C.

Beispiel A

Tabletten können aus den folgenden Bestandteilen in konventioneller Weise hergestellt werden:

| Bestandteile | pro Tablette |
|---|---|
| Verbindung der Formel I | 5 mg |
| Lactose | 125 mg |
| Maisstärke | 65 mg |
| Talk | 4 mg |
| Magnesiumstearat | 1 mg |
| Tablettengewicht | 200 mg |

Beispiel B

Eine Füllmenge für Gelatinekapseln kann aus folgenden Bestandteilen hergestellt werden:

| Bestandteile | | pro Kapsel |
|---|---|---|
| Verbindung der Formel I | | 10 mg |
| Lactose | | 165 mg |
| Maisstärke | | 20 mg |
| Talk | | 5 mg |
| | Kapselfüllgewicht | 200 mg |

## Patentansprüche

1. Purinderivate der allgemeinen Formel

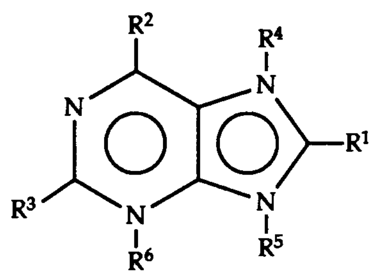

I

worin
$R^1$ Wasserstoff oder nieder-Alkyl;
$R^2$ Amino, Mono- oder Di-(nieder-alkyl)amino, $-SR^1$ oder $-OR^1$;
$R^3$ Wasserstoff, Amino, Mono- oder Di(nieder-alkyl)amino, $-SR^1$ oder $-OR^1$;
$R^4$, $R^5$ und $R^6$ einen Rest der Formel

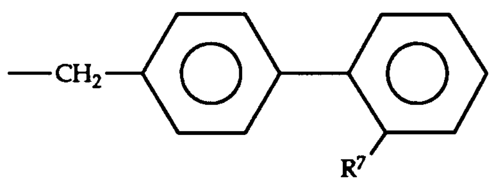

(a)

$R^7$ die Gruppe $-COOR^8$ oder einen Tetrazolylrest der Formel

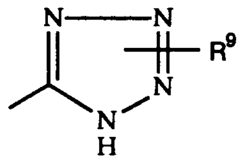

(b)

$R^8$ Wasserstoff, ein Alkali-, Erdalkali- oder Ammonium-Kation, nieder-Alkyl oder einen unter physiologischen Bedingungen abspaltbaren Rest; und
$R^9$ Wasserstoff oder ein Alkali-, Erdalkali- oder Ammonium-Kation bedeuten; wobei nur einer der Substituenten $R^4$, $R^5$ und $R^6$ anwesend ist und $R^2$ Amino oder Mono- oder Di-(nieder-alkyl)amino bedeutet, wenn $R^6$ anwesend ist.

2. Verbindungen gemäss Anspruch 1 der allgemeinen Formel

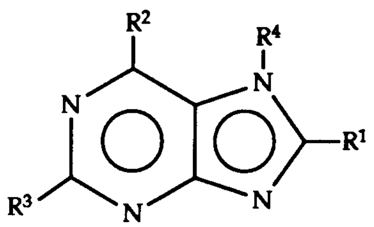

I a

worin $R^1$, $R^2$, $R^3$ und $R^4$ die in Anspruch 1 angegebene Bedeutung haben.

**3.** Verbindungen gemäss Anspruch 1 der allgemeinen Formel

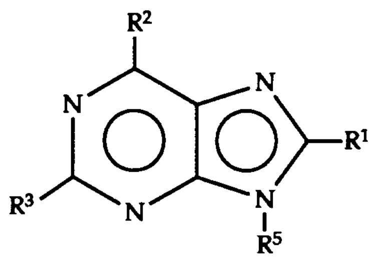

I b

worin $R^1$, $R^2$, $R^3$ und $R^5$ die in Anspruch 1 angegebene Bedeutung haben.

**4.** Verbindungen gemäss Anspruch 1 der allgemeinen Formel

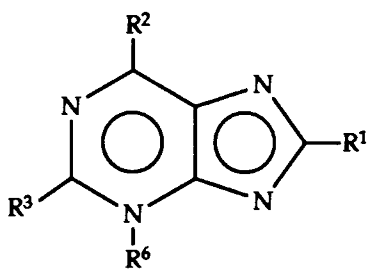

I c

worin $R^1$, $R^2$, $R^3$ und $R^6$ die in Anspruch 1 angegebene Bedeutung haben.

**5.** Verbindungen gemäss einem der Ansprüche 1-4, worin $R^7$ einen Tetrazolylrest der Formel (b) bedeutet.

**6.** Verbindungen gemäss einem der Ansprüche 1-5, worin $R^1$ nieder-Alkyl, insbesondere n-Propyl oder n-Butyl, bedeutet.

**7.** 4'-[(2-Amino-1,6-dihydro-6-oxo-9H-purin-9-yl)methyl]-2-biphenylcarbonsäure,
4'-[(2-Amino-1,6-dihydro-6-oxo-7H-purin-7-yl)methyl]-2-biphenylcarbonsäure,
4'-[(8-Butyl-1,6-dihydro-6-oxo-9H-purin-9-yl)methyl]-2-biphenylcarbonsäure,
4'-[(1,6-Dihydro-6-oxo-8-propyl-9H-purin-9-yl)methyl]-2-biphenylcarbonsäure,
4'-[(8-Butyl-6-methoxy-7H-purin-7-yl)methyl]-2-biphenylcarbonsäure,
4'-[(6-Amino-8-butyl-3H-purin-3-yl)methyl]-2-biphenylcarbonsäure,
4'-[(1,6-Dihydro-6-oxo-7H-purin-7-yl)methyl]-2-biphenylcarbonsäure,
Methyl 4'-[(6-amino-8-butyl-9H-purin-9-yl)methyl]-2-biphenylcarboxylat,
Methyl 4'-[(6-amino-8-butyl-3H-purin-3-yl)methyl]-2-biphenylcarboxylat,
Methyl 4'-[(6-methoxy-8-propyl-9H-purin-9-yl)methyl]-2-biphenylcarboxylat,
Methyl 4'-[(6-methoxy-8-propyl-7H-purin-7-yl)methyl]-2-biphenylcarboxylat,

Methyl 4'-[(1,6-dihydro-6-oxo-7H-purin-7-yl)methyl]-2-biphenylcarboxylat,
Methyl 4'-[(2-acetamido-6-[(diphenylcarbamoyl)oxy]-9H-purin-9-yl)methyl]-2-biphenylcarboxylat oder
Methyl 4'-[(2-acetamido-6-[(diphenylcarbamoyl)oxy]-7H-purin-7-yl)methyl]-2-biphenylcarboxylat.

**8.** 4'-[(8-Butyl-1,6-dihydro-6-oxo-7H-purin-7-yl)methyl]-2-biphenylcarbonsäure.

**9.** 4'-[(1,6-Dihydro-6-oxo-8-propyl-7H-purin-7-yl)methyl]-2-biphenylcarbonsäure.

**10.** 4'-[(8-Butyl-6-methoxy-9H-purin-9-yl)methyl]-2-biphenylcarbonsäure.

**11.** 4'-[(6-Amino-8-butyl-9H-purin-9-yl)methyl]-2-biphenylcarbonsäure.

**12.** 8-Butyl-1,9-dihydro-9-[[2'-(1H-tetrazol-5-yl)-4-biphenylyl]methyl]-6H-purin-6-on.

**13.** 8-Butyl-1,7-dihydro-7-[[2'-(1H-tetrazol-5-yl)-4-biphenylyl]methyl]-6H-purin-6-on.

**14.** Purinderivate gemäss einem der Ansprüche 1-13 zur Anwendung als therapeutische Wirkstoffe.

**15.** Purinderivate gemäss einem der Ansprüche 1-13 zur Anwendung bei der Bekämpfung bzw. Verhütung von Bluthochdruck und Arteriosklerose.

**16.** Verfahren zur Herstellung einer Verbindung gemäss einem der Ansprüche 1-13, dadurch gekennzeichnet, dass man eine Verbindung der allgemeinen Formel

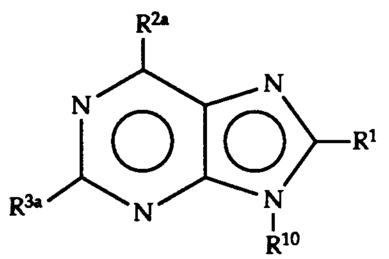

II

worin $R^{2a}$ einen wie in Anspruch 1 definierten Rest $R^2$ bedeutet, wobei die Amino-, Mercapto- oder Hydroxygruppe in geschützter Form vorliegen kann; $R^{3a}$ einen wie in Anspruch 1 definierten Rest $R^3$ bedeutet, wobei die Amino-, Mercapto- oder Hydroxygruppe in geschützter Form vorliegen kann; $R^{10}$ Wasserstoff oder ein N,N- oder N,O-Ketal bzw. -Aminal bedeutet; und $R^1$ die in Anspruch 1 angegebene Bedeutung hat;
mit einer Verbindung der allgemeinenFormel

Z-X III

worin X eine Abgangsgruppe und Z einen Rest der Formel

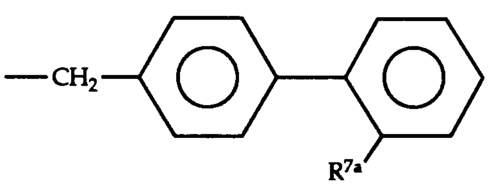

(a')

$R^{7a}$ die Gruppe $-COOR^{8a}$ oder einen Tetrazolylrest der Formel

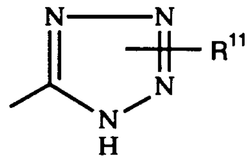

(b')

$R^{8a}$ nieder-Alkyl oder einen unter physiologischen Bedingungen abspaltbaren Rest und $R^{11}$ eine Schutzgruppe bedeuten, umsetzt, aus dem Reaktionsprodukt gegebenenfalls anwesende Schutzgruppen abspaltet und erwünschtenfalls eine Estergruppe $R^{8a}$ verseift, und eine Aether- oder Thioäthergruppe $R^2$ spaltet und/oder erwünschtenfalls das Reaktionsprodukt in ein Salz überführt.

**17.** Arzneimittel, enthaltend ein Purinderivat gemäss einem der Ansprüche 1-13 und ein therapeutisch inertes Excipiens.

**18.** Mittel zur Bekämpfung bzw. Verhütung von Bluthochdruck und Arteriosklerose, enthaltend ein Purinderivat gemäss einem der Ansprüche 1-13 und ein therapeutisch inertes Excipiens.

**19.** Verwendung eines Purinderivates gemäss einem der Ansprüche 1-13 zur Herstellung von Mitteln gegen Bluthochdruck und/oder Arteriosklerose.

**Patentansprüche für folgende Vertragsstaaten: GR, ES**

**1.** Verfahren zur Herstellung von Purinderivaten der allgemeinen Formel

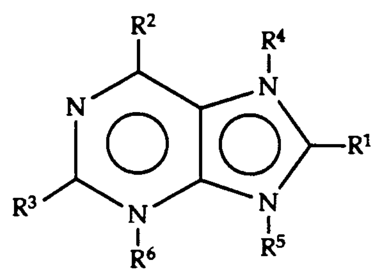

I

worin
$R^1$ Wasserstoff oder nieder-Alkyl;
$R^2$ Amino, Mono- oder Di-(nieder-alkyl)amino, $-SR^1$ oder $-OR^1$;
$R^3$ Wasserstoff, Amino, Mono- oder Di(nieder-alkyl)amino, $-SR^1$ oder $-OR^1$;
$R^4$, $R^5$ und $R^6$ einen Rest der Formel

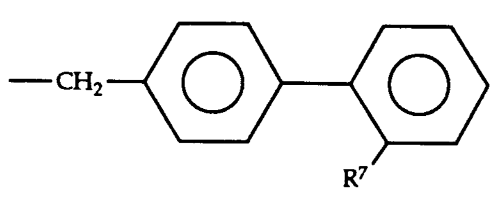

(a)

$R^7$ die Gruppe $-COOR^8$ oder einen Tetrazolylrest der Formel

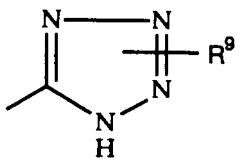

(b)

$R^8$ Wasserstoff, ein Alkali-, Erdalkali- oder Ammonium-Kation, nieder-Alkyl oder einen unter physiologischen Bedingungen abspaltbaren Rest; und

$R^9$ Wasserstoff oder ein Alkali-, Erdalkali- oder Ammonium-Kation bedeuten; wobei nur einer der Substituenten $R^4$, $R^5$ und $R^6$ anwesend ist und $R^2$ Amino oder Mono- oder Di-(nieder-alkyl)amino bedeutet, wenn $R^6$ anwesend ist,

dadurch gekennzeichnet, dass man eine Verbindung der allgemeinen Formel

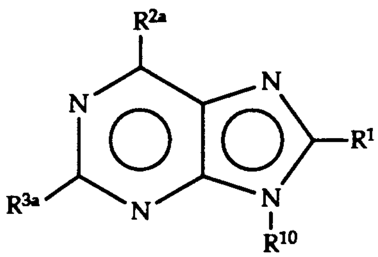

II

worin $R^{2a}$ einen wie oben definierten Rest $R^2$ bedeutet, wobei die Amino-, Mercapto- oder Hydroxygruppe in geschützter Form vorliegen kann; $R^{3a}$ einen wie oben definierten Rest $R^3$ bedeutet, wobei die Amino-, Mercapto- oder Hydroxygruppe in geschützter Form vorliegen kann; $R^{10}$ Wasserstoff oder ein N,N- oder N,O-Ketal bzw. -Aminal bedeutet; und $R^1$ die oben angegebene Bedeutung hat; mit einer Verbindung der allgemeinenFormel

Z-X III

worin X eine Abgangsgruppe und Z einen Rest der Formel

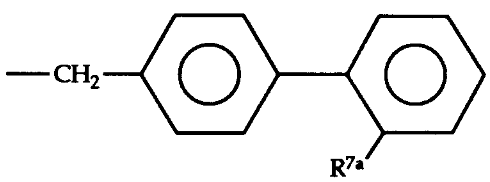

(a')

$R^{7a}$ die Gruppe $-COOR^{8a}$ oder einen Tetrazolylrest der Formel

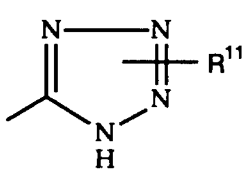

(b')

$R^{8a}$ nieder-Alkyl oder einen unter physiologischen Bedingungen abspaltbaren Rest und $R^{11}$ eine Schutzgruppe bedeuten,

umsetzt, aus dem Reaktionsprodukt gegebenenfalls anwesende Schutzgruppen abspaltet und erwünschtenfalls eine Estergruppe $R^{8a}$ verseift, und eine Aether- oder Thioäthergruppe $R^2$ spaltet und/oder erwünschtenfalls das Reaktionsprodukt in ein Salz überführt.

**2.** Verfahren gemäss Anspruch 1, dadurch gekennzeichnet, dass man eine Verbindung der allgemeinen Formel

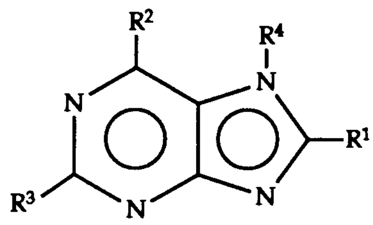

I a

worin $R^1$, $R^2$, $R^3$ und $R^4$ die in Anspruch 1 angegebene Bedeutung haben, herstellt.

**3.** Verfahren gemäss Anspruch 1, dadurch gekennzeichnet, dass man eine Verbindung der allgemeinen Formel

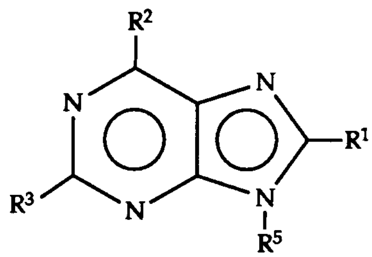

I b

worin $R^1$, $R^2$, $R^3$ und $R^5$ die in Anspruch 1 angegebene Bedeutung haben, herstellt.

**4.** Verfahren gemäss Anspruch 1, dadurch gekennzeichnet, dass man eine Verbindung der allgemeinen Formel

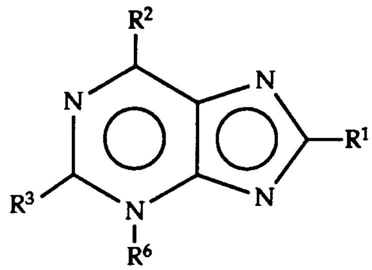

I c

worin $R^1$, $R^2$, $R^3$ und $R^6$ die in Anspruch 1 angegebene Bedeutung haben, herstellt.

**5.** Verfahren gemäss einem der Ansprüche 1-4, worin $R^7$ einen Tetrazolylrest der Formel (n) bedeutet.

**6.** Verfahren gemäss einem der Ansprüche 1-5, worin $R^1$ nieder-Alkyl, insbesondere n-Propyl oder n-Butyl, bedeutet.

**7.** Verfahren gemäss Anspruch 1, dadurch gekennzeichnet, dass man 4'-[(8-Butyl-1,6-dihydro-6-oxo-7H-purin-7-yl)methyl]-2-biphenylcarbonsäure herstellt.

**8.** Verfahren gemäss Anspruch 1, dadurch gekennzeichnet, dass man 4'-[(1,6-Dihydro-6-oxo-8-propyl-7H-purin-7-yl)methyl]-2-biphenylcarbonsäure herstellt.

**9.** Verfahren gemäss Anspruch 1, dadurch gekennzeichnet, dass man 4'-[(8-Butyl-6-methoxy-9H-purin-9-yl)methyl]-2-biphenylcarbonsäure herstellt.

**10.** Verfahren gemäss Anspruch 1, dadurch gekennzeichnet, dass man 4'-[(6-Amino-8-butyl-9H-purin-9-yl)-methyl]-2-biphenylcarbonsäure herstellt.

**11.** Verfahren gemäss Anspruch 1, dadurch gekennzeichnet, dass man 8-Butyl-1,9-dihydro-9-[[2'-(1H-tetrazol-5-yl)-4-biphenylyl]methyl]-6H-purin-6-on herstellt.

**12.** Verfahren gemäss Anspruch 1, dadurch gekennzeichnet, dass man 8-Butyl-1,7-dihydro-7-[[2'-(1H-tetrazol-5-yl)-4-biphenylyl]methyl]-6H-purin-6-on herstellt.

**13.** Verfahren zur Herstellung von Arzneimitteln, insbesondere zur Verwendung bei der Bekämpfung bzw. Verhütung von Bluthochdruck und Arteriosklerose, dadurch gekennzeichnet, dass man ein Purinderivat der Formel I in Anspruch 1 in eine galenische Darreichungsform bringt.

**14.** Verwendung eines Purinderivates der Formel I in Anspruch 1 zur Herstellung von Mitteln gegen Bluthochdruck und/oder Arteriosklerose.